# EUROPEAN PATENT APPLICATION

(11) **EP 2 532 646 A1**
(43) Date of publication of application: **12.12.2012**
(21) Application number: 12170844.0
(22) Date of filing: 05.06.2012
(51) Int. Cl.: C07C 67/11, C07C 69/14, C07C 69/16, C07C 69/157

(54) **Process for preparing esters and organic halides**

(30) Priority: 07.06.2011 EP 11168952
(71) Applicant: BASF SE, 67056 Ludwigshafen (DE); Universität Siegen Institut für Anorganische Chemie, 57068 Siegen (DE)
(72) Inventor: Massonne, Klemens, 67098 Bad Dürkheim (DE); Siemer, Michael, 68159 Mannheim (DE); Mormann, Werner, 57223 Kreuztal (DE); Frank, Petra, 57223 Kreuztal (DE)

(57) **Abstract**

A process for preparing esters and organic halides, which comprises reacting
- a salt having a melting point of less than 100°C (at 1 bar) and of the formula

(K⁺)ₙ X (COO-)ₙ,

in which
K⁺ is an organic cation,
X (COO-)ₙ is an organic anion having an n-valent organic group X which is substituted by n carboxylate groups COO-, and
n is 1, 2 or 3,
- with an organic halogen compound (Hal)ₘY,
in which
Hal is a halogen atom,
Y is an m-valent organic group, and
m is 1, 2 or 3,
to give an ester and a halide K⁺ Hal-.

## Description

This patent application claims the benefit of pending US provisional patent application Serial Number 61/494003 filed June 7, 2011 incorporated in its entirety herein by reference.

The present invention relates to a process for preparing esters and organic halides, which comprises reacting
- a salt having a melting point of less than 100°C (at 1 bar) and of the formula

   (K⁺)ₙX(COO⁻)ₙ,

   in which
   K⁺ is an organic cation,
   X(COO⁻)ₙ is an organic anion having an n-valent organic group X which is substituted by n carboxylate groups COO⁻, and
   n is 1, 2 or 3,
- with an organic halogen compound (Hal)ₘY,
   in which
   Hal is a halogen atom,
   Y is an m-valent organic group, and
   m is 1, 2 or 3,
to give an ester and a halide K⁺ Hal⁻.

Organic esters and organic halides are of diverse importance for industrial chemistry. They find use for a very wide variety of industrial applications, or serve as starting materials for the synthesis of chemical compounds. Of particular interest are organic halides which are liquid at room temperature, examples being imidazolium halides. Salts which are liquid at room temperature are also referred to as ionic liquids.

There is therefore a fundamental need for simple and cost-effective syntheses for organic esters and organic halides.

"Methoden der organischen Chemie", Houben-Weyl, 4th edition, volume VIII, pages 541 to 543 and DE-A 24 03 445 disclose an ester synthesis in which a metal salt with a carboxylate anion and an organic halogen compound are used as starting material. In analogy to the Williamson synthesis for ethers, the products of this synthesis are an organic ester and a metal halide.

L. Brinchi, R. Germani, and G. Savelli, Tetrahedron Letters 44 (2003) 2027-2029, and James McNulty et al, Tetrahedron Letters 46 (2005) 3641-3644, disclose the use of ionic liquids as solvents in esterifications.

The object of the present invention was an extremely simple and effective process for preparing organic esters. Reference may be made here more particularly also to oligomeric or polymeric esters, whose preparation generally entails increased cost and complexity.

Another object of the present invention was an extremely simple and effective process for preparing organic halides, including in particular those organic halides which are ionic liquids and therefore have particular industrial importance.

Accordingly, the process defined at the outset has been found. Also found, more particularly, have been a process for preparing oligomeric or polymeric esters and a process for preparing ionic liquids with organic cations and a halide as anion.

In the process of the invention, a salt having a melting point of less than 100°C (at 1 bar) and of the formula

(K⁺)ₙX(COO⁻)ₙ,

in which
K⁺ is an organic cation,
X(COO⁻)ₙ is an organic anion having an n-valent organic group X which is substituted by n carboxylate groups COO⁻, and
n is 1, 2 or 3,
is reacted with an organic halogen compound (Hal)ₘY,
in which
Hal is a halogen atom,
Y is an m-valent organic group, and
m is 1, 2 or 3,
to give an ester and a halide K⁺ Hal⁻.

### The cation K⁺

The cation K⁺ is preferably a quaternary ammonium cation or a cation having a nitrogen-containing ring system.

The cation K⁺ preferably contains no nitrogen atom substituted by a proton.

Preferred quaternary ammonium cations are those having four aliphatic substituents, more preferably C1 to C12 alkyl groups, on the nitrogen atom, it being possible, optionally, for these substituents to be substituted by hydroxyl groups.

Likewise preferred are organic cations which comprise a heterocyclic ring system, with at least one, preferably one to three, nitrogen atom(s) being part of the ring system.

Ring systems contemplated include monocyclic, bicyclic, aromatic or nonaromatic ring systems. Examples include bicyclic systems, as described in WO 2008/043837. The bicyclic systems of WO 2008/043837 are diazabicyclo derivatives, preferably composed of a 7-membered ring and of a 6-membered ring, which comprise an amidinium group; mention may be made more particularly of the 1,8-diazabicyclo[5.4.0]undec-7-enium cation.

Especially preferred are cations which comprise a heterocyclic ring system having one or two nitrogen atoms as part of the ring system.

Examples of suitable such organic cations include pyridinium cations, pyridazinium cations, pyrimidinium cations, pyrazinium cations, imidazolium cations, pyrazolium cations, pyrazolinium cations, imidazolinium cations, thiazolium cations, triazolium cations, pyrrolidinium cations, and imidazolidinium cations. These cations are recited in WO 2005/113702, for example. Where necessary for a positive charge on the nitrogen atom or in the aromatic ring system, the nitrogen atoms are each substituted by an organic group having in general not more than 20 C atoms, preferably a hydrocarbon group, more particularly a C1 to C16 alkyl group, more particularly a C1 to C10, very preferably a C1 to C4 alkyl group.

The carbon atoms of the ring system as well may be substituted by organic groups having in general not more than 20 C atoms, preferably a hydrocarbon group, more particularly a C1 to C16 alkyl group, more particularly a C1 to C10, very preferably a C1 to C4 alkyl group.

Particularly preferred ammonium cations are quaternary ammonium cations, imidazolium cations, pyrimidinium cations, and pyrazolium cations.

With particular preference the organic cation K⁺ is an imidazolium cation of the formula I below, 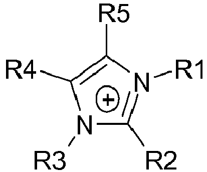 in which

R1 and R3 independently of one another are an organic radical having 1 to 20 C atoms and R2, R4 and R5 are an H atom or an organic radical having 1 to 20 C atoms.

In formula I, R1 and R3 are preferably, independently, an organic radical having 1 to 10 C atoms. More particularly, R1 and R3 are an aliphatic radical, more particularly an aliphatic radical without further heteroatoms, and are, for example, an alkyl group. With particular preference, R1 and R3 independently of one another are a C1 to C10, more particularly a C1 to C4 alkyl group.

In formula I, R2, R4, and R5 are preferably, independently, an H atom or an organic radical having 1 to 10 C atoms; more particularly, R2, R4, and R5 are an H atom or an aliphatic radical. With particular preference, R2, R4, and R5 independently of one another are an H atom or an alkyl group, and more particularly R2, R4, and R5 independently of one another are an H atom or a C1 to C4 alkyl group. With very particular preference, R2, R4, and R5 are each an H atom.

### The anion

Depending on whether n is 1, 2 or 3, the anion X(COO⁻)ₙ is an anion having one, two or three carboxylate groups.

Preferably, n is 1 or 2. In one preferred embodiment of the invention n is 1.

X is an n-valent organic group, preferably an organic group having 1 to 20, more particularly 1 to 10 C atoms.

More preferably, X is an n-valent hydrocarbon radical having 1 to 20 C atoms, more particularly 1 to 10 C atoms.

More preferably, X is a C1 to C10 alkyl radical (n=1) or is a phenyl radical (n=1) or a C2 to C10 alkylene radical (n=2) or a phenylene radical (n=2).

As preferred anions, mention may be made more particularly of formate, acetate, propionate, butanoate, hexanoate, octanoate, benzoate, phthalates (phthalate, isophthalate, terephthalate), 1,2-ethylenedicarboxylate, 1,4-butylenedicarboxylate, 1,6-hexylenedicarboxylate.

In one preferred embodiment, the salt (K⁺)ₙ X(COO⁻)ₙ is a salt in which

K⁺ is an imidazolium cation of the formula I and

X(COO⁻)ₙ is an organic anion, X being an n-valent hydrocarbon radical having 1 to 20 C atoms, more particularly 1 to 10 C atoms, and n being 1 or 2.

The halogen compound (Hal)ₘY

Hal is a halogen atom, more particularly a chlorine or bromine atom.

Preferably, m is 1 or 2. In one preferred embodiment of the invention m is 1.

Y is an n-valent organic group, preferably an organic group having 1 to 20, more particularly 1 to 10 C atoms.

More preferably, Y is an n-valent hydrocarbon radical having 1 to 20 C atoms, more particularly 1 to 10 C atoms.

More preferably, Y is a C1 to C10 alkyl radical (m=1) or is a phenyl radical +(m=1) or a C2 to C10 alkylene radical (m=2) or a phenylene radical (m=2).

### Products

The reaction produces an ester and an organic halide K⁺ Hal⁻.

Different esters are formed according to the value of n in (K⁺)ₙX(COO⁻)ₙ and of m in (Hal)ₘY.

If m is 1, an ester of the formula X(COOY)ₙ is obtained, n being 1, 2 or 3.

If n is 1, an ester of the formula (XCOO)ₘY is obtained, m being 1, 2 or 3.

If n and m are 1, an ester of the formula XCOOY is obtained.

If n and m are 2, an ester having repeating units (XCOOYCOO) is obtained.

Preference is given to esters XCOOY (n=1 and m=1) and also to oligomeric esters having repeating units XCOOYCOO (n=2 and m=2).

It is an advantage of the process of the invention that oligomeric or polymeric esters having repeating units (XCOOYCOO) are obtainable. The oligomeric and polymeric esters may be obtained with high molar weights, as for example with molar weights of more than 5000, also in particular with molar weights of more than 10 000 and with molar weights of more than 20 000 g/mol (weight average Mw).

The halide obtained through the reaction, K⁺ Hal⁻, is a salt which, depending on the cation, may also have a melting point of less than 100°C (1 bar, atmospheric pressure). With the above-recited preferred cations (quaternary ammonium cation or a cation having a nitrogen-containing ring system, especially, for example, imidazolium of the formula I), the K⁺ Hal⁻ halides obtained are generally ionic liquids. In the process of the invention, these ionic liquids are produced in high purity; for numerous industrial applications of ionic liquids, this is of great importance.

In one particular embodiment, therefore, the process of the invention is an advantageous process for preparing an ionic liquid, i.e., for preparing halides having a melting point of less than 100°C (1 bar), and more particularly, for example, for preparing imidazolium chlorides, the imidazolium cation conforming more particularly to formula I above.

The implementation of the process of the invention

Starting materials are the salt having a melting point of less than 100°C (at 1 bar) and of the formula (K⁺)ₙX(COO⁻)ₙ, and the organic halogen compound (Hal)ₘY.

The two starting materials are used advantageously in amounts that as far as possible are stoichiometric, taking account of the values of n and m, in order that both starting materials react completely, as far as possible, and there is no need for subsequent removal of unreacted starting material. Where rational or advantageous for industrial implementation of the process, however, it is also possible for one of the starting materials to be used in excess.

The reaction may be carried out under reduced or increased pressure; the reaction is generally performed simply under atmospheric pressure. The reaction takes place preferably at elevated temperature, more particularly at temperatures of 30 to 200°C, more particularly 40 to 150°C, very preferably at 50 to 100°C.

Accompanying use of solvent is unnecessary, since the salt employed is an ionic liquid. The halogen compounds used are also generally liquid under the reaction conditions.

The ester obtained in the reaction, and the halide K⁺ Hal⁻ obtained, are also generally liquid, and in particular the halide obtained is in turn an ionic liquid. After the reaction, advantageously, the ester and the halide are frequently obtained as two separate liquid phases and can in that case easily be separated by means, for example, of the removal of one phase by decanting. Particularly if chlorine compounds are used as starting material (organic halogen compound), two separate liquid phases are generally obtained.

If the ester obtained and the halide obtained are miscible and therefore only one liquid phase is obtained, the mixture can be separated by conventional techniques - for example, one of the two products can be separated off by extraction, using a suitable extractant, such as an ether such as diethyl ether, for example, or by distillation, such as, for example, distilling of the ester from the mixture.

The process of the invention is a simple and effective process for preparing esters, including oligomeric or polymeric esters. The esters are obtained in high purity and yield.

Correspondingly, the process of the invention is also a simple and effective process for preparing halides, more particularly ionic liquids with a halide anion. The halides and ionic liquids are obtained in high purity and yield.

### Examples

### Starting materials

EMIM-Acetate is the salt 1-Ethyl-3-methylimidazolium acetate (see cation of formula I with R1=ethyl and R3=methyl)
BMIM-CL is the salt 1-Butyl-3-methylimidazolium-chloride (see cation of formula I with R1=butyl and R3=methyl)
(MIM)₂ adipate is the salt 1-Methylimidazolium (two moles)- adipate (one mol) (see cation of formula I with R1= methyl and R3=H)
(EMIM)₂ -adipate the salt 1-Ethyl-3-methylimidazolium (two moles)- adipate (one mol) (see cation of formula I with R1= ethyl and R3=methyl)
(EMI M)₂-terephthalate the salt 1-Ethyl-3-methylimidazolium (two moles)- therephthalate (one mol) (see cation of formula I with R1= ethyl and R3=methyl)

### Synthesis of (EMIM)₂ -adipate

A 1 L round bottom flask was charged with approximately 250 g (0.4 mol) of 30 % EMIM-methylcarbonate in methanol (the exact content has been determined by addition of excess sulfuric acid and titration with NaOH). The equivalent amount (0.2 mol) of adipic acid dissolved in 150 mL methanol was added dropwise at room temperature under stirring while formation of carbon dioxide gas was observed. When no more gas evolved volatiles were removed initially at 60°C, 12 hPa, finally at 90°C, 0,05 hPa.

¹H NMR (CD₃CN, 400.13 MHz): δ = 1.37 (t, 3H, CH₃), 1.91 (t, 3H, CH₂CO), 3.89 (s, 3H, CH₃), 4.22 (q, 2H, CH₂), 7.64 & 7.70 (s, 2H, =CH), 10.73 (CH=N⁺) ¹³C NMR: δ = 15.9, 28.8, 36.4, 40.6, 45.3, 122.9, 124.5, 140.0, 178.6; FTIR (ATR mode): *v̅* = 2937 (br), 1566 (s), 1175 (m)

### Synthesis of (EMIM)₂-terephthalate

(EMIM)₂-terephthalate was made as described for the adipate replacing adipic acid by terephthalic acid, which was added as a solid. When no more gas evolved volatiles were removed initially at 60°C, 12 hPa, finally at 90°C, 0,05 hPa.

### Synthesis of (Mim)₂-adipate

A 100 mL round bottom flask equipped with a magnetic stirring bar and methylimidazole (18.0.0g, 0.220 mol) and 0.5 equivalents of adipic acid (16.0 g, 0.11 mol) were added at room temperature, while adding adipic acid exothermic was observed, the reaction was stirred for 20h at 90°C. The reaction mixture was cooled to rt, pure product was obtained as a solid (99.0% yield).

¹H NMR (CD₃CN, 400.13 MHz): δ = 1.58 (m, 4H, CH₂), 2.26 (m, 4H, CH₂), 3.67 (s, 3H, CH₃), 7.0 (d, 2H, =CH), 7.64 (s, 2H, NH), 12.76 (CH=N⁺) ¹³C NMR: δ = 25.4, 34.0, 34.9, 121.7, 127.6, 138.5, 176.6; FTIR (ATR mode): *v̅* = 3133 (br), 2945 (br), 1700 (m), 1083 (m). MP: 83.3°C.

### Analytical measurements:

NMR data were obtained at 400.13 MHz on a Bruker Spectrospin 400 and are listed in parts per million (ppm) relative to tetramethylsilane. IR spectra were recorded on a Bruker Equinox 55 FT-IR spectrometer using ATR technology.

Gel permeation chromatography of the polymeric adipates (examples 10 to 15) was performed in tetrahydrofuran. A calibrated SDV-1000 Å column was used with a separation capacity of 600 to 60.000 Dalton polystyrene. For comparison a polybutylenadipate with a molecular weight of 2000 was used. The polybutylenadipate (FRP458) had a weight average molecular weight Mw of 2000 which corresponds to a degree of polymerization (DP) of 10.

### General remarks to examples 1 - 7

All procedures were performed under dry nitrogen atmosphere or by standard Schlenk technique.
EMIM-Acetate can be used as efficient reagent for the esterification reaction of aliphatic halogen compounds by simply exchanging the anion of the respective compounds (scheme 1). The direct reaction was carried out between the corresponding alkyl halide and EMIM-Acetate in equimolar ratios. The reaction was fast, exothermic and proceeded in either homogeneous or in a two-phase system.

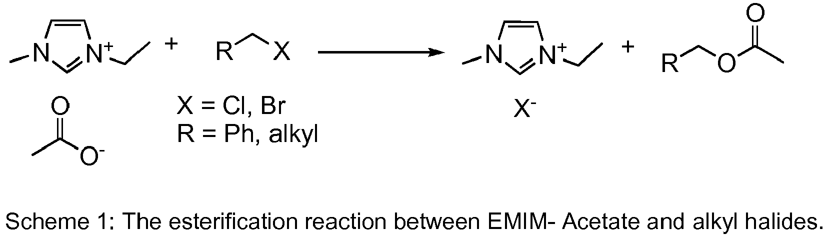

Esterification with chloride compounds mostly proceeds in a two-phase system whereas bromoides react in a homogeneous system. In heterogeneous systems the ester product usually forms the upper layer. Products are isolated in homogeneous systems by extraction with diethyl ether, in two phase systems by separation (decanting) of the upper layer. Yields obtained were in the range from 82-95.0%.
The obtained salts EMIM-Cl or EMIM-Br (lower layer) are ionic liquids. They were dried under vacuum to remove volatiles and were analytically pure.

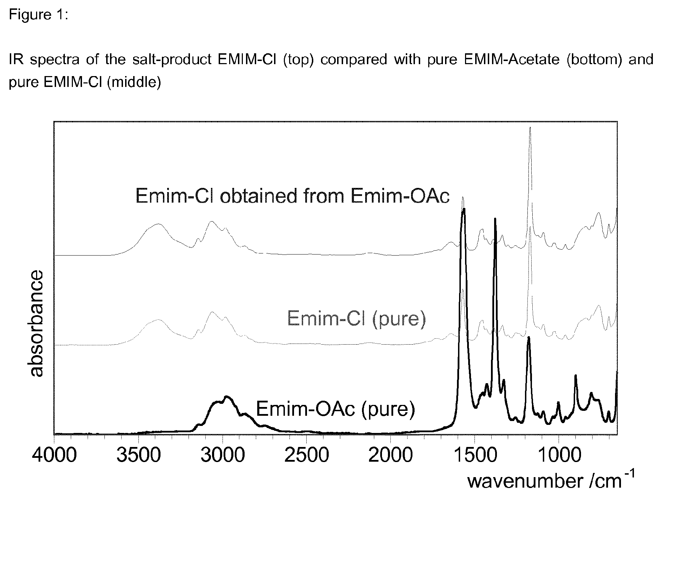

In order to show the purity of the obtained EMIM-Cl, the lower layer was washed with diethyl ether as well. The EMIM-Cl was compared with the starting EMIM-Acetate and commercial, pure EMIM-Cl (Figure 1). No traces of EMIM-Acetate were found, hence it was completely converted to EMIM-Cl. The IR- spectrum of the obtained EMIM-Cl corresponds exactly to that of pure EMIM-Cl. Hence EMIM-CL synthesized this way was pure and even better as compared to the commercial one in terms of colour.

**Table 1:**

| | | | | | |
|---|---|---|---|---|---|
| Summary of investigations of EMIM-acetate with different types of halide comp (T=reaction time; T= temperature) | | | | | |

| **Example. No** | **Substrate** | **t/h** | **T/°C** | **Yield (%)** | **Product** |
|---|---|---|---|---|---|
| 1 | 1,2-Dichloroethane | 3 | 70 | 90 | 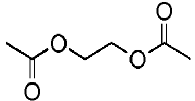 |
| 2 | 1,2-Dibromoethane | 3 | 20 | 90 | |
| 3 | Dibromomethane | 2 | 20 | 85 | 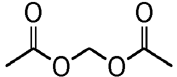 |
| 4 | Dichloromethane | 16 | 20 | 86 | |
| 5 | Benzyl chloride | 2 | 20 | 95 | 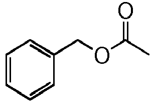 |
| 6 | 2-Chlorobutane | 16 | 80 | 82 | 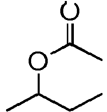 |
| 7 | 2-Bromobutane | 16 | 20 | 83 | |

### Example 1 and 2: ethylene glycol diacetate

A 100 mL Schlenk flask was equipped with a magnetic stirring bar, EMIM-acetate (50.0 g, 0.293 mol), and 0.5 equivalents of 1,2-dichloroethane (14.5 g, 0.146 mol) were added at room temperature and reaction was stirred at 70°C for 3h. After reaction two phases were obtained upper layer was decanted and the reaction mixture was extracted 3 times with diethyl ether, all fractions were combined and diethyl ether was removed under vacuum. The final pure product was obtained as color less liquid (90.0% yield)
When 1, 2-dibromoethane was used as substrate the reaction was very fast even at room temperature and was highly exothermic. The reaction proceeds via homogeneous pathway; the pure product was distilled from the reaction mixture at 100°C under reduced pressure (90.0% yield).

Ethylene glycol diacetate: ¹H NMR (CDCl₃, 400.13 MHz): δ = 2.02 (s, 6 H, CH₃), 4.2 (s, 4H, CH₂); ¹³C NMR: δ = 20.9 (CH₃), 62.3 (CH₂), 170.9 (CO); FTIR (ATR mode): *v̅* = 2961 (br), 1735 (s), 1371 (m), 1213 (s), 1048 (m).

### Examples 3 and 4: methylene diacetate

A 50 mL Schlenk flask was equipped with a magnetic stirring bar, EMIM-acetate (20.0 g, 117.0 mmol), and 0.5 equivalents of dichloromethane (5.0 g, 58.7 mmol) were added at room temperature and the reaction was stirred at 50°C for 16h. After reaction the pure Product was distilled from the reaction mixture at 80°C under reduced pressure (85.0-%).
When dibromomethane was used as substrate the reaction was fast even at room temperature and highly exothermic. The reaction proceeds via homogeneous path way and the pure product distilled from the reaction mixture at 80°C under reduced pressure (86.0% yield).
Methylene diacetate: ¹H NMR (CD₃CN, 400.13 MHz): δ = 2.04 (s, 6H, CH₃), 5.64 (s, 2H, CH₂); ¹³C NMR: δ = 20.9 (CH₃), 79.7 (CH₂), 170.8 (CO); FTIR (ATR mode): *v̅* = 2996 (br), 1758 (s), 1190 (s), 1009 (s), 978 (s)

### Example 5: benzyl acetate

A 100 mL Schlenk flask was equipped with a magnetic stirring bar, EMIM-acetate (40.0 g, 0.235 mol), and benzyl chloride (28.0 g, 0.221 mol) were added at room temperature and reaction mixture was stirred for 2h at room temperature (rt), the reaction was fast and exothermic. After reaction two phases were obtained, the desired product formed the upper layer and was decanted (95.0% yield).

¹H NMR (CDCl₃, 400.13 MHz): δ = 2.09 (s, 3H, CH₃), 5.1 (s, 2H, CH₂), 7.35 (m, 5H, Ph); ¹³C NMR: δ = 21.2 (CH₃), 66.5 (CH₂), 122.4, 128.7, 136.1(Ph), 171.0 (CO); FTIR (ATR mode): *v̅* = 3034 (br), 1736 (s), 1222 (s), 1025 (s).

### Examples 6 and 7: sec-butyl acetate

A 50 mL Schlenk flask was equipped with a magnetic stirring bar, EMIM-acetate (20.0 g, 0.117 mol), and 1.0 equivalents of 2-bromobutane (15.9 g, 0.116 mmol) were added at room temperature and the reaction was stirred at room temperature for 16h. After reaction two phases were obtained and the upper layer (product) was decanted (83.0% yield).
When 2-chlorobutane was used as substrate the reaction was stirred at 80°C for 16h. After reaction two phases were obtained. To get the pure product upper layer was decanted (82.0% yield).

¹H NMR (CD₃CN, 400.13 MHz): δ = 0.87 (t, 3H, CH₃), 1.15 (d, 3H, CH₃), 1.53 (m, 2H, CH₂), 4.75 (m, 1H, CH) ¹³C NMR: δ = 10.0 (CH₃), 19.8 (CH₃), 21.5 (CH₃), 29.6 (CH₂), 72.8 (CH), 171.3 (CO); FTIR (ATR mode): *v̅* = 2974 (br), 1733 (s), 1371 (m), 1238 (s), 1030 (m).

### Example 8: benzyl benzoate

In a similar way one reaction was investigated with EMIM-benzoate as esterification reagent with benzyl chloride (Scheme 2). The reaction results in a two phase system, benzyl benzoate was isolated in a quantitative yield (95.0-%).

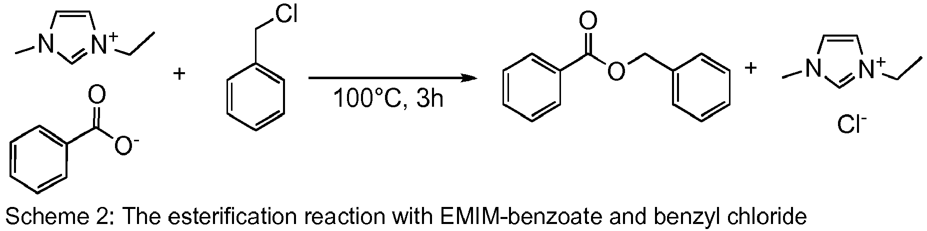

### Example 9: Methyl (R)- (+)-2-acetoxy propionate

An optically pure halide compound which is methyl-(R)-(+)-2- chloro propionate and EMIM-acetate were used as starting compounds (Scheme 3).

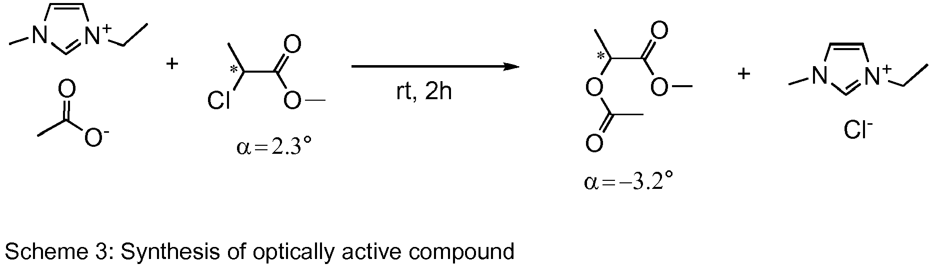

A 25 mL Schlenk flask was equipped with a magnetic stirring bar, EMIM-acetate (1.0 g, 5.875 mmol), and 1.0 equivalents of methyl (R)- (+)-2-chloro propionate (0.72 g, 5.875mmol) were added at room temperature and reaction was stirred at room temperature for 2h. After the reaction, two phases were obtained upper layer was decanted which was contained pure compound (90.0% yield).

¹H NMR (CDCl₃, 400.13 MHz): δ = 1.46 (d, 3H, CH₃), 1.12 (s, 3H, CH₃), 3.74 (s, 3H, CH₃), 5.06 (q, 1H, CH); FTIR (ATR mode): *v̅* = 1738 (s), 1372 (m), 1207 (m), 1097 (m).

Optical activity: Optical activity of the starting material as well as product was measured with polarimeter. Sample was prepared in 20 mL heptane by dissolving 0.7g of methyl (R)- (+)-2-chloro propanate. The solution of 0.7 g of methyl (R)- (+)-2-acetoxy propionate was prepared in the same solvent. The specific rotation was found to be (α) = -3.20 at room temperature, whereas that of the starting material was 2.3°.

### Examples 10 to 17: polymeric esters

### Polymer synthesis (general procedure)

A flame dried 25 mL Schlenck flask with magnetic stirring bar was charged with 5-7 g (EMIM)₂ - adipate or (EMIM)₂ -terephthalate, to which was added the exact stoichiometric amount of α,α'-dichlor-p-xylene or 1,4-dichlorobutane or dichloroethane. Mixtures were heated to the temperature T and for the time t. Polymers were isolated by precipitation in methanol or dispersion in methanol with a high speed stirrer (terephthalates). Precipitates were collected and dried at 60°C, 0.05 hPa. Details are listed in table 2.

Polymer from adipic acid and dichloro-p-xylene:

(EMIM)₂ -adipate was prepared with 99.0-% yield from the reaction between EMIM-methyl-carbonate and adipic acid. (EMIM)₂ -adipate was used as monomer like adipic acid and it reacted with dichloro-p-xylene (which is also named 1,4-Bis(chloromethyl)benzene) or 1,2-dichloroethane at 90°C and 70°C for 20h respectively (185 & 187). The reaction mixture was precipitated with methanol. The degree of polymerization was calculated from NMR as well as from GPC. The GPC data is shown in table 2.

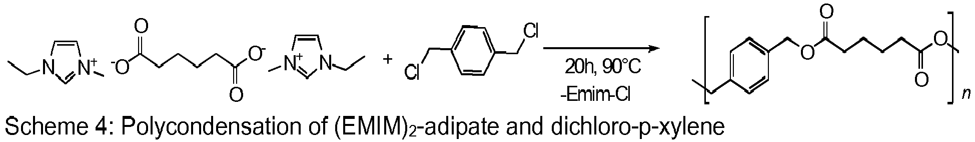

In addition MIM)₂-adipate was synthesized by neutralization of adipic acid with methylimidazol (MIM) and a polymerization was attempted by the reaction of (MIM)₂-adipate and dichloro-p-xylene. In this case, no reaction occurred even at 100°C (example 11).

**Table 2: examples 10 to 17, compounds and results**

| Example No. | Imidazolium salt | | halogen compound | | | t(°C)/ T (h) | Yield (%) | | *Mw*/*Mn* |
|---|---|---|---|---|---|---|---|---|---|
| 10 | (EMIM)₂-adipate | | 1,2-dichloro ethane | | | 70/20 | 75 | | 9600/17200 |
| 11 | (Mim)₂-adipate | | 1,4-Bis(chloromethyl) benzene | | | 100/16 | 0 | | 0 |
| | | | | | | | | | |

| Poly-p-xylen-adipate from (EMIM)₂-adipate and dichloro-p-xylene molecular weight of repeating unit 248 g/mol | | | | | | | | | |
|---|---|---|---|---|---|---|---|---|---|
| Example No. | T /°C t /h | yield. /% | Solubility in | | | | | NMR | GPC |
| | | | | | | | m.p. /°C | DP | Mw /DP |
| 12 | 90 / 20 | 97 | CHCl₃, THF | | | | 81,3 / 87,3 | 50 | 23500 / 95 |
| 13 | 130 / 96 | 87 | CHCl₃, THF | | | | 77,6 / 85,0 79 / 86 | 40 | 17500 / 70 |
| 14 | 140 / 72 | 50 | CHCl₃, THF | | | | 84,2 | n.d. (130) | 24000 / 97 |

| Poly-butylen-adipate from (EMIM)₂ adipate and dichlorobutane molecular weight of repeating unit 192 g/mol | | | | | | | | | |
|---|---|---|---|---|---|---|---|---|---|
| 15 | 120/168 | 25 | CHCl₃, THF | | | | 35,3 / 42,8 | 2 | 2000 / 10 |

| Poly-p-xylen-terephthalate from (EMIM)₂ terephthalate and dichloro-p-xylene Molecular weight of repeating unit 268,26 g/mol | | | | | | | | | |
|---|---|---|---|---|---|---|---|---|---|
| 16 | 140 / 24 | 70 | DMSO (hot), CHCl₃/ TFA | | | | 273,4 Zers. ab 300; 271² | 4 | n.d. |

| Poly-butylen-terephthalate from (EMIM)₂ terephthalate and dichlorobutane Molecular weight of repeating unit 220,22 g/mol | | | | | | | | | |
|---|---|---|---|---|---|---|---|---|---|
| 17 | 130 / 24 | 83 | DMSO (hot), TCE¹, CHCl₃/ TFA | | | | 217,0 Zers. ab 242; 218 | | n.d. |

| | | | | | | | | | |
|---|---|---|---|---|---|---|---|---|---|
| n.d.: not determined m.p.: melting point | | | | | | | | | |

## Claims

1. A process for preparing esters and organic halides, which comprises reacting
- a salt having a melting point of less than 100°C (at 1 bar) and of the formula
(K⁺)ₙ X (COO⁻)ₙ,
in which
K⁺ is an organic cation,
X (COO⁻)ₙ is an organic anion having an n-valent organic group X which is substituted by n carboxylate groups COO⁻, and
n is 1, 2 or 3,
- with an organic halogen compound (Hal)ₘY,
in which
Hal is a halogen atom,
Y is an m-valent organic group, and
m is 1, 2 or 3,
to give an ester and a halide K⁺ Hal⁻.

2. The process according to claim 1, wherein the cation K⁺ is a quaternary ammonium cation or a cation having a nitrogen-containing ring system.

3. The process according to either of claims 1 and 2, wherein the cation contains no nitrogen atom substituted by a proton.

4. The process according to any of claims 1 to 3, wherein the cation K⁺ is an imidazolium cation of the formula I below, 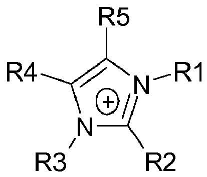 in which
R1 and R3 independently of one another are an organic radical having 1 to 20 C atoms and
R2, R4, and R5 are an H atom or an organic radical having 1 to 20 C atoms.

5. The process according to any of claims 1 to 4, wherein X is a hydrocarbon radical having 1 to 20 C atoms .

6. The process according to claim 5, wherein X is a C1 to C10 alkyl radical (n=1) or is a phenyl radical (n=1) or a C2 to C10 alkylene radical (n=2) or a phenylene radical (n=2).

7. The process according to any of claims 1 to 6, wherein the salt (K⁺)ₙX(COO⁻)ₙ is a salt in which
K⁺ is an imidazolium cation of the formula I and
X(COO⁻)ₙ is an organic anion, X being an n-valent hydrocarbon radical having 1 to 20 C atoms and n being 1 or 2.

8. The process according to any of claims 1 to 7, wherein Hal is a chlorine or bromine atom.

9. The process according to any of claims 1 to 8, wherein Y is a hydrocarbon radical having 1 to 20 C atoms.

10. The process according to claim 9, wherein Y is a C1 to C10 alkyl radical (m=1) or is a phenyl radical (m=1) or a C2 to C10 alkylene radical (m=2) or a phenylene radical (m=2).

11. The process according to any of claims 1 to 10, wherein the ester obtained by the reaction
is an ester of the formula X(COOY)ₙ if m is 1,
or is an ester of the formula (XCOO)ₘY, if n is 1,
or is an ester of the formula XCOOY, if n and m are 1,
or is an ester having repeating units (XCOOYCOO), if n and m are 2.

12. The process according to any of claims 1 to 11, wherein the resulting halide K⁺ Hal⁻ is an ionic liquid, i.e., has a melting point of less than 100°C (1 bar).

13. The process according to claim 12, wherein the resulting halide K⁺ Hal⁻ is an imidazolium chloride.

14. The process according to any of claims 1 to 13, wherein the process is carried out without use of additional solvent.

15. The process according to any of claims 1 to 14, wherein the resulting ester and the resulting halide K⁺ Hal⁻ are liquid and are obtained as separate liquid phases.
